# EUROPEAN PATENT SPECIFICATION

(11) **EP 0 289 992 B1**
(45) Date of publication and mention of the grant of the patent: **27.04.1994**
(21) Application number: 88107103.9
(22) Date of filing: 03.05.1988
(51) Int. Cl.: C07D 473/00

(54) **Process for preparing purine derivates**
Verfahren zu Herstellung von Purinverbindungen
Procédé pour la préparation de dérivés de purine

(30) Priority: 04.05.1987 YU 786/87; 06.11.1987 YU 2020/87
(43) Date of publication of application: 09.11.1988
(60) Divisional application: 93108617.7
(73) Proprietor: KEMIJSKI INSTITUT, SI-61000 Ljubljana (SI)
(72) Inventor: Kobe, Joze, YU-61000 Ljubljana (YU); Stimac, Anton, YU-61000 Ljubljana (YU); Zupet, Pavle, YU-68000 Novo mesto (YU); Gnidovec, Joze, YU-68000 Novo mesto (YU); Plavec, Janez, YU-68000 Novo mesto (YU)
(74) Representative: Müller-Boré & Partner Patentanwälte

(56) References cited:
- EP-A- 0 108 285
- DE-A- 3 544 461
- GB-A- 1 567 671
- US-A- 4 146 715

## Description

The present invention relates to a process for preparing purine derivatives of the formula I 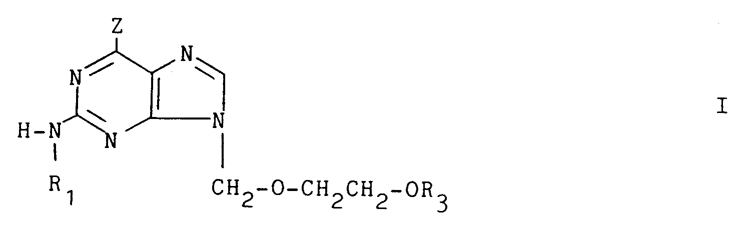
wherein
- R₁: represents hydrogen, CH₃CO or a higher acyl,
- R₃: represents hydrogen, CH₃CO or a higher acyl, CF₃CO, CCl₃CO, PhCO or CH₂ Ar, such as benzyl or substituted benzyl, and benzoisothiazolyl-S,S-dioxide, and
- Z: represents OH, hydrogen or halo.
wherein the following compounds are excluded:
(i) Z is OH, R₁ is CH₃CO or higher acyl, and R₃ is hydrogen,
(ii) Z is hydrogen, R₁ is CH₃CO or higher acyl, and R₃ is hydrogen, and
(iii) Z is halo, R₁ is CH₃CO or higher acyl, and R₃ is hydrogen
The compounds of the formula I represent - depending upon the different values of the radicals - either intermediates for deoxyacyclovir and acyclovir or deoxyacyclovir (Z = R₁= R₃= H) and acyclovir (Z = OH, R₁= R₃= H) themselves. Acyclovir is the leading medicine for the treatment of herpes infections (Zovirax). Deoxyacyclovir, which is a prodrug of acyclovir, is by means of the enzyme xanthinoxidase quickly converted to acyclovir "in vivo", the "in vivo" activity of the latter being several times greater than the activity of formulated acyclovir (i.e. acyclovir in the form of pharmaceutical formulation).

The synthesis of one of the known compounds of the formula I, i.e. deoxyacyclovir, is disclosed in literature (T.A. Krenitsky et al, Proc. Natl. Acad. Sci., USA (1984) 81, 3209-3213, GB 2 130 204A), preferably via the intermediary compound 2-amino-6-chloro-9-(2-benzoyloxythoxymethyl)purine with dehalogenation and deblocking, or by direct condensation from aminopurine and suitably activated and protected side-chain.

The synthesis of another known compound of formula I , i.e. acyclovir, is disclosed in literature (H.J. Schaeffer et al, (1978) Nature (London) 272, 583-585, US 4,360,522), preferably via guanine.

From the same literature there is also evident the synthesis of the compound I (R₁= H, R₃= CH₃CO, Z = OH) from acyclovir. J. Kobe et al (US 4,701,526) also teaches the synthesis of acyclovir from guanine, yet via guanine-glyoxal-adduct to the key intermediate 9-(2-acetoxyethoxymethyl)-glyoxal N²-acetyl-guanine adduct III (R = R₁= R₃= CH₃CO).

The process for preparing compounds of the formula I is carried out in such a way that
a) according to the first variant there are prepared the compounds of the formula I, wherein Z represents halo or hydrogen and R₁ is hydrogen, from the protected acyclovir of the formula II 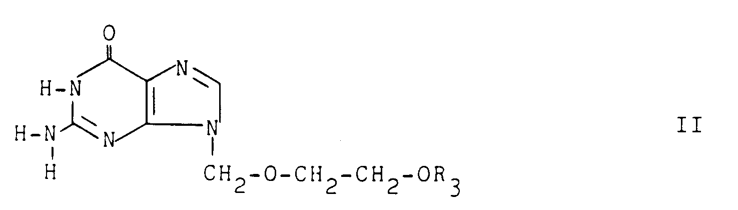 wherein R₃ has the above meanings except hydrogen, by the reaction thereof with a halogenating agent, such as POCl₃, in the presence of tetraethyl ammonium chloride and N,N-dimethylaniline in acetonitrile at reflux, to obtain the compound of the formula I with Z = halo, such as chloro, and R₃ having the above meanings except hydrogen, which compound is hydrogenated and, when R₃ is different from CH₂Ar or substituted aryl, is deblocked to the compound I with Z = hydrogen and R₃ = hydrogen.
   The compounds of formula II are prepared according to conventional methods (T.W. Green, Protective Groups in Organic Synthesis), when R₃ is different from H. Some of these compounds (R₃ is CF₃CO, CCl₃CO, benzoisothiazolyl-S,S-dioxide) are novel compounds.
   As already mentioned, derivatives of 6-chloropurine (a compound of the formula I, wherein Z = Cl and R₃ is different from hydrogen) are by hydrogenation converted to the protected deoxyacyclovir (Z = H, R₃≠ H, CH₂Ar, subst. aryl) and then deblocked (R₃= H) in a weak basic medium, e.g. with ammonia, methylamine (50 %), NaOMe etc., at room temperature. For particular protective groups the deblocking is carried out under conditions disclosed in literature (T.W. Greene, Protective Groups in Organic Synthesis).
   When R₃ is CH₂Ar or substituted Ar, the hydrogenation alone directly, i.e. without subsequent deblocking, gives unprotected deoxyacyclovir, e.g. catalytic dehydrogenation with Pd/C 5 %.
   In the process variant a) according to the invention, the selection of the starting material i.e. adequately protected acyclovir of the formula II, obtained e.g. for R₃= H according to the process in US 4,701,526, is very convenient. Said protected acyclovir is in a simple way and with good yields converted to known 2-amino-6-chloro-9-(2-benzoyloxyethoxymethyl)purine or to novel 2-amino-6-chloro-9-(2-subst. oxyethoxymethyl)purines, which by catalytic hydrogenation may be converted into the prodrug of acyclovir, i.e.deoxyacyclovir.
   The process according to the invention may also be carried out in such a way that
b) according to the second variant there are prepared compounds of the formula I with all meanings of radicals as given in the introductory in such a way that a compound of the formula III 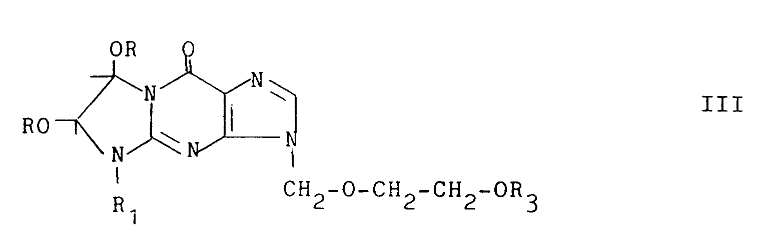 wherein R represents CH₃CO, isobutyryl or higher acyl and R₁ and R₃ have the above meanings except hydrogen, is selectively deblocked with weak bases or acids or in the presence of a catalyst to a compound of the formula I, wherein Z is OH, R₁ has the meaning at formula I and R₃ has the meaning at formula I except hydrogen, which compound is optionally reacted as in the process variant a), i.e. the obtained compound is halogenated to obtain a compound of the formula I, wherein Z is halo, and the latter is, by hydrogenation or photochemical dehalogenation, optionally converted to compound I, wherein Z is hydrogen.

As weak bases or acids diluted mineral and organic acids and bases are suitable.

As catalysts there can be used triethanolamine, methylamine, imidazole, silicagel, molecular sieves, sodium methoxide.

By halogenation of compounds of the formula I, wherein Z is OH, R₁ has the meanings at formula I and R₃ also has the meanings at formula I except hydrogen, with e.g. POCl₃ in the presence or absence of tetraethylammonium chloride and in the presence of N,N-dialkylanilines, preferably diethylaniline, and also of pyridine in acetonitrile at reflux, there is obtained a compound I (Z = Cl), which is by hydrogenation or photochemical dehalogenation converted to protected deoxyacyclovir (Z = H, R₁ and R₃ have the meanings given at formula I except hydrogen, or one of them should not be hydrogen). Alternatively, compounds of the formula I, wherein Z is OH, R₁ is H and R₃ has the meanings given at formula I except benzyl, substituted aryl or hydrogen, are by deblocking in a basic medium, e.g. with ammonia, methylamine (50 %), NaOMe, NaOH, at room temperature, converted to acyclovir (Z = OH, R₁= R₃= H). In the case of compound I (Z = Cl, R₁= acetyl, R₃= benzyl), for direct synthesis of deoxyacyclovir prodrug or for direct synthesis of deoxyacyclovir there are used hydrogenation technique or other general methods for deblocking under conditions disclosed in literature (T.W. Greene, Protective Groups in Organic Synthesis).

Due to a convenient combination of the meanings of R₁ and R₃ there exist novel possibilities for further chemical or enzymatic conversions of OH-group as Z in 6-position into other groups, e.g. preferably halo, but also amino, thio, alkylthio, lower alkoxy, azido.

The process variant b) according to the invention makes possible a direct synthesis of the intermediate I, i.e. adequately protected acyclovir, from the compound of formula III disclosed in US 4,701,526, and not from acyclovir (US 4,360,522).

The process variant b) has one step more than a), yet on the other hand, the product represents a pure isomer and according to said variant there are also available two novel compounds as an enrichment of the art.

Some derivatives of formula I are in a simple way and with good yields converted to acyclovir or to already known 2-amino-6-chloro-9-(2-acetoxyethoxymethyl)purine or also to novel 2-amino-6-chloro-9-(2-subst. oxyethoxymethyl)purines, which may subsequently, by means of catalytic hydrogenation or photochemical dehalogenation, be converted into a prodrug of acyclovir, i.e. deoxyacyclovir.

The invention is illustrated in detail by the following Examples.

### Example 1 (variant a)

### 9-(2-acetoxyethoxymethyl)guanine

9-(2-hydroxyethoxymethyl)guanine (acyclovir) (13.8 g), dry dimethyl formamide (140 ml), acetanhydride (48 ml) and dry pyridine (72 ml) were stirred overnight at room temperature. The suspension was filtered, a white residue was dissolved in hot dimethyl formamide (300 ml), subsequently pyridine (30 ml) and acetanhydride (24 ml) were added and it was stirred for 18 hours at room temperature. White crystals of 9-(2-acetoxyethoxymethyl)guanine were filtered, recrystallized from dimethyl formamide, washed with ethyl acetate and dried in vacuo at 110°C.
Yield:10.04 g (61 %), m.p. 234-245°C.

### Example 2 (variant a)

### 9-(2-acetoxyethoxymethyl)guanine

9-(2-hydroxyethoxymethy1)-N²-acetylguanine (150 mg, 0.5 mmole) was heated under stirring at reflux for 7 hours in methanol (5 ml) with silicagel (0.5 g; Kieselgel 60 HF₂₅₄, Merck), previously dried at 110°C for 1 hour. The boiling suspension was filtered through Celite and washed with hot methanol (25 ml). After evaporation crude 9-(2-acetoxyethoxymethyl)guanine (75 mg; 57 %) was obtained, m.p. 241-242°C.

### Example 3 (variant b)

### 9-(2-acetoxyethoxymethyl)guanine

To a solution of 9-(2-acetoxyethoxymethyl)-diacetoxy-glyoxal-N²-acetylguanine-adduct of the formula III (R = R₁= R₃= CH₃CO; 0.5 g, 1.1 mmole) with the chemical name 5-acetyl-6,7-diacetoxy-3-(2-acetoxyethoxymethyl)-5,6,7,9-tetrahydro-3H-imidazo/1,2-a/purine-9-one in hot methanol (20 ml) there was added triethanolamine (1.64 g, 11 mmole) and the reaction mixture was heated at reflux for 5 hours. After cooling to room temperature 9-(2-acetoxyethoxymethyl)guanine (75 g) of the formula I (R₁= H, R₃= CH₃CO, Z = OH) was obtained. The mother liquor was evaporated and water (5 ml) was added to the residue. There was obtained the compound of the formula I (100 mg; R₁= H, R₃= CH₃CO, Z = OH). The total yield was 175 mg (59 %), m.p. 240-242°C. The product was identical with the compound disclosed in U.S. patent 4,360,522.

### Example 4 (variant b)

### N²-acetyl-9-(2-acetoxyethoxymethyl)guanine

To a solution of 9-(2-acetoxyethoxymethyl)-diacetoxyglyoxal-N²-acetylguanine-adduct of the formula III (R = R₁= R₃= CH₃CO; 45 mg, 0.1 mmole) in hot methanol (2 ml) 2 drops (∼50 mg, 0.3 mmole) of triethanolamine were added and the mixture was heated for 70 minutes at reflux. Water (1 ml) was added to the evaporated mixture. A compound of m.p. 203-205°C 10 mg; 33 %) was obtained, which was identified as N²-acetyl-9-(2-acetoxyethoxymethyl)guanine of the formula I (R₁= R₃= CH₃CO, Z = OH).
¹H NMR - DMSO-d₆, δ-TMS
1.95 (s, 3, OCO Me)
2.17 (s, 3, NNCO Me)
3.71 (m, 2, OCH₂CH₂)
4.06 (m, 2, OCH₂CH₂)
5.46 (s, 2, NCH₂O)
8.10 (s, 1, H₈)
11.7 (broad. s, NH)
12.0 (broad. s, NH)
The aqueous liquor was evaporated and the residue (yellow oil) was re-dissolved in hot methanol. After cooling there was obtained 9-(2-acetoxyethoxymethyl)guanine (10 mg; 38 %) of the formula I (R₁ = H, R₃= CH₃CO, Z = OH).

### Example 5 (variant b)

### 9-(2-acetoxyethoxymethyl)guanine

a) N²-acetyl-9-(2-acetoxyethoxymethyl)guanine (15.5 g, 0.05 mole) and triethanolamine (7.5 g, 0.05 mole) were heated at reflux for 2.5 hours under stirring in methanol (750 ml). The reaction mixture was left to cool to room temperature, the obtained crystals were filtered and washed with methanol (cca 100 ml) to a neutral pH. After air-drying there was obtained 9-(2-acetoxyethoxymethyl)guanine (12.23 g; 92 %), m.p. 242-244°C (H.J. Schaeffer, US 4,287,188).
¹H NMR - DMSO-d₆, δ-TMS
1.95 (s, 3, COCH₃)
3.66 (m, 2, OCH₂CH₂OAc)
4.05 (m, 2, OCH₂CH₂OAc)
5.34 (s, 2, NCH₂O)
6.49 (broad s, 2, NH₂)
7.79 (s, 1, H₈)
10.65 (broad s, 1, NH)

| Elemental analysis: | | | |
|---|---|---|---|
| Calc.: | C 44.94 | H 4.90 | N 26.20 |
| Found: | C 45.00 | H 4.90 | N 26.39 |

### Example 6 (variant b)

### 9-(2-acetoxyethoxymethyl)-2-amino-9H-purine

a) The crude residue (1.08 g) from the reaction of the example 10 hereinafter containing 9-(2-acetoxyethoxymethyl)-2-amino-6-chloro-9H-purine (0.5 g) was dissolved in absolute ethanol (100 ml), triethylamine (1 ml) and 10 % Pd on charcoal (0.5 g) were added and hydrogenation was carried out for 15 hours in a Parr hydrogenator at an initial pressure of 350000 Pa (50 psi) and at room temperature. The mixture was filtered and the ethanolic solution was evaporated to a solid residue, which was extracted with boiling benzene (3 x 50 ml). The combined extracts were evaporated to an oily residue, from which the product was isolated by chromatography on silicagel (80 g; 70 to 230 mesh). First the impurities were washed with etyhl acetate (cca 2 l) and then 9-(2-acetoxyethoxymethyl)-2-amino-9H-purine (0.30 g, 68 %) was washed with acetone (cca 1 l).
b) 9-(2-acetoxyethoxymethyl)-2-amino-6-chloro-9H-purine (71 mg, 0.25 mmole) was dissolved in dry tetrahydrofuran (18 ml) and triethylamine (2 ml). The solution was flushed with nitrogen for 10 minutes and then photolyzed in a photochemical reactor Rayonet (254 nm). The volatile components were evaporated, the residue was dissolved in chloroform (5 ml) and washed with water (3 x 40 ml). The organic layer was dried with anhydrous sodium sulfate and the solvent was evaporated. There was obtained crude 9-(2-acetoxyethoxymethyl)-2-amino-9H-purine (34mg), identical to the product obtained by method a).

### Example 7 (variant b)

### 2-amino-9-(2-hydroxyethoxymethyl)-9H-purine (DEOXYACYCLOVIR)

9-(2-acetoxyethoxymethyl)-2-amino-9H-purine (0.3 g) was stirred for 1 hour with a 40 % aqueous methylamine solution (2 ml) and ethanol (5 ml). The volatile components were evaporated to a solid residue and crystallized from ethanol. There was obtained pure 2-amino-9-(2-hydroxyethoxymethyl)-9H-purine (102 mg, 41 %), m.p. 189-192°C.
¹H NMR - DMSO-d₆, δ-DSS
3.50 (s, 4, CH₂CH₂)
4.66 (s, 1, OH)
5.45 (s, 2, NCH₂O)
6.53 (s, 2, NH₂)
8.13 (s, 1, H₈)
8.56 (s, 1, H₆)

### Example 8

A. A mixture of 9-(2-acetoxyethoxymethyl)-N²-acetyl guanine (618 mg, 2 mmole), silicagel powder (2.50 g; Kieselgel 60 HF₂₅₄, Merck) and methanol (30 ml) was vigorously stirred at reflux for 8 hours. The solvent was evaporated and the resulting solid was boiled for 1 minute with dimethyl formamide (30 ml). The suspension was filtered through Celite, the layer of silica was extracted with boiling dimethyl formamide (10 ml), filtered through Celite and washed with hot solvent (5 ml). Combined filtrates were evaporated to yield 9-(2-acetoxyethoxymethyl)guanine (515mg, 96 %), m.p. 242-244°C, which is identical with the product obtained in method A. The crude product contained max. 10 % of acyclovir according to HPLC analysis.
B. A mixture of 9-(2-acetoxyethoxymethyl)-N²-acetyl guanine (618 mg, 2 mmole), molecular sieve 0.3 nm powder (618 mg, Molekularsieb 3 Å Pulver, Merck) and methanol (30 ml) was vigirously stirred at reflux for 2 hours. The solvent was evaporated, the resulting solid was heated in dimethyl formamide (30 ml) to the boiling point, quickly filtered through Celite and washed with hot dimethyl formamide (2 X 5 ml). The filtrate was evaporated to yield 9-(2-acetoxyethoxymethyl)guanine (534 mg, 100 %), m.p. 238-242°C, which is identical with the product obtained in method A. The crude product contained about 15 % of acyclovir according to HPLC analysis.
C. A mixture of 9-(2-acetoxyethoxymethyl)-N²-acetyl guanine (1.55 g, 5 mmole), imidazole (0.34 g), methanol (30 ml) and water (10 ml) was stirred at reflux for 16 hours. The obtained crystalline product was filtered while hot, successively washed with methanol, water and methanol, and dried to give 9-(2-acetoxyethoxymethyl)guanine (0.65 g, 48 %), m.p. 242-243°C, which is identical with the product obtained in method A. The crude product contained max. 6 % of acyclovir according to HPLC analysis.

### Example 10

### 9-(2-acetoxyethoxymethyl)-2-amino-6-chloro-9H-purine

A solution of 9-(2-acetoxyethoxymethyl)guanine (4.01 g, 15 mmole), tetraethylammonium chloride (4.98 g, 30 mmole; dried in vacuo at 85°C overnight over P₂O₅), N,N-dimethylaniline (1.92 ml, 15 mmole; dried over and distilled from CaH₂) and freshly distilled phosphorus oxychloride (8.4 ml, 90 mmole) in acetonitrile (30 ml) was heated with stirring at reflux for 15 minutes. The volatile materials were evaporated in vacuo, the resulting oil was dissolved in chloroform (90 ml) and vigorously stirred with broken ice for 15 minutes. The layers were separated and the aqueous pahse was extracted with chloroform (6 x 50 ml). The combined organic phase was washed with cold water (6 x 50 ml) and 5 % NaHCO₃/H₂O (50 ml), dried over Na₂SO₄ overnight and filtered. The evaporation gave a crude product (2.06 g), which was applied to a silicagel column and eluted first with chloroform (1 l) to remove N,N-dimethylaniline and then with ethyl acetate. The fractions containing the required product free of by-products (TLC in EtOAc: R_{f}= 0.35) were combined and evaporated to give 9-(2-acetoxyethoxymethyl)-2-amino-6-chloro-9H-purine (0.88g, 21 %) with m.p. 104-105°C after recrystallization first from ethyl acetate and then from 2-propanol.
¹H NMR (CDCl₃) δ-TMS:
2.04 (s, 3, COCH₃)
3.76 (A₂B₂m, 2, OCH₂CH₂OAc)
4.21 (A₂B₂m, 2, OCH₂CH₂OAc)
5.53 (s, 2, OCH₂Base)
5.63 (br, 2, NH₂)
7.93 (s, 1, H₈)
ms m/z: 285 (M⁺)

### Example 11

### 9-(2-acetoxyethoxymethyl)-2-acetamido-6-chloro-9H-purine

A solution of 9-(2-acetoxyethoxymethyl)-N²-acetylguanine (3.09 g, 10 mmole), N,N-diethylaniline (1.59 ml, 10 mmole) and freshly distilled phosphorus oxychloride (9.6 ml) in acetonitrile (40 ml) was heated with stirring at reflux for 30 minutes. The volatile materials were evaporated in vacuo, ice (50 g) was added to the residue and it was extracted with chloroform (10 x 30 ml). The combined organic phase was washed with cold water (3 x 75 ml and 5 % NaHCO₃/H₂O (100 ml), dried over Na₂SO₄ for 1 hour and filtered. The evaporation gave an orange viscous substance (1.31 g), which was applied to a silicagel column and the column was eluted with acetone (300 ml). A crude product (0.67 g) was obtained, which was repurified on a second silicagel column and eluted with 1:1 benzene:ethyl acetate to yield 9-(2-acetoxyethoxymethyl)-2-acetamido-6-chloro-9H-purine (0.47 g, 14 %) with m.p. 150-150.5°C after recrystallization from ethyl acetate.
¹H NMR - DMSO-d₆, δ-TMS:
1.96 (s, 3, OCOCH₃)
2.26 (s, 3, NHCOCH₃)
3.83 (A₂B₂m, 2, OCH₂CH₂OAc)
4.13 (A₂B₂m, 2, OCH₂CH₂OAc)
5.68 (s, 2, OCH₂Base)
8.69 (s, 1, H₈)
10.75 (br, 1, NH)

### Example 12

### 9-(2-acetoxyethoxymethyl)-2-amino-9H-purine

To a solution of 9-(2-acetoxyethoxymethyl)-2-amino-6-chloro-9H-purine (7.15 g, 25 mmole) in acetone (100 ml) there were succesively added triethylamine (35 ml, 250 mmole), formic acid (5 ml, 125 mmole) and 10 % palladium on charcoal (1 g). The mixture was heated with vigorous stirring at reflux for 1 hour, filtered while hot and the catalyst was washed with boiling acetone. The filtrate was evaporated to dryness, the residue was dissolved in water (100-150 ml) and the solution was continuously extracted with dichloromethane overnight. The organic layer was dried over Na₂SO₄ for cca 1 hour and evaporated to dryness to give a crude product (6.25 g). It was recrystallized from 100 % ethanol to give 9-(2-acetoxyethoxymethyl)-2-amino-9H-purine (5.51 g, 88 %) with m.p. 133-133.5°C.
¹H NMR (CDCl₃) δ:
2.03 (s, 3, COCH₃)
3.75 (A₂B₂m, 2, OCH₂CH₂OAc)
4.17 (A₂B₂m, 2, OCH₂CH₂OAc)
5.52 (s, 4, OCH₂Base and NH₂)
7.88 (s, 1, H₈)
8.68 (s, 1, H₆)

### Example 13

### 9-(2-acetoxyethoxymethyl)-2-acetamido-9H-purine

To a mixture of 9-(2-acetoxyethoxymethyl)-2-acetamido-6-chloro-9H-purine (3.28 g, 10 mmole) and acetone (40 ml), there were successively added triethylamine (14 ml, 100 mmole), formic acid (2 ml, 50 mmole) and 10 % palladium on charcoal (0.4 g). The mixture was heated with vigorous stirring at reflux for 1 hour, filtered while hot and the catalyst was washed with boiling acetone and chloroform. The filtrate was evaporated to dryness, the residue was dissolved in water (150 ml) and the solution was continuously extracted with dichloromethane overnight. The organic layer was dried over Na₂SO₄ for cca 1 hour and evaporated to dryness to give a crude product (2.78 g). It was recrystallized from 100 % ethanol to give 9-(2-acetoxyethoxymethyl)-2-acetamido-9H-purine (2.56 g, 87 %) with m.p. 134.5-135.5°C.
¹H NMR (CDCl₃) δ-TMS:
2.02 (s, 3, OCOCH₃)
2.57 (s, 3, NHCOCH₃)
3.80 (A₂B₂m, 2, OCH₂CH₂OAc)
4.18 (A₂B₂m, 2, OCH₂CH₂OAc)
5.66 (s, 2, OCH₂Base)
8.15 (s, 1, H₈)
9.07 (s, 1, H₆)
9.95 (br, 1, NH)

### Example 14

### 2-amino-9-(2-hydroxyethoxymethyl)-9H-purine (DEOXYACYCLOVIR)

A. 9-(2-acetoxyethoxymethyl)-2-amino-9H-purine (5.02 g, 20 mmole) was dissolved in water (50 ml), 40 % aqueous methylamine (25 ml) was added and the solution was stirred at room temperature for 10 minutes. The reaction mixture was evaporated to dryness with successive addition of water and ethanol. The crude product was recrystallized from 100 % ethanol with the addition of activated charcoal to give deoxyacyclovir (3.41 g, 81.5 %) as analytically pure white crystals with m.p. 192.5-193°C.
¹H NMR - DMSO-d₆, δ-DSS:
3.50 (s, 4, CH₂CH₂)
4.70 (s, 1, OH)
5.50 (s, 2, OCH₂Base)
6.63 (s, 2, NH₂)
8.20 (s, 1, H₈)
8.63 (s, 1, H₆)
ms m/z: 209 (M⁺)

| Elemental analysis: | | | |
|---|---|---|---|
| Calc.: | C 45.93 | H 5.30 | N 33.47 |
| Found: | C 45.38 | H 5.19 | N 33.89 |

B. 9-(2-acetoxyethoxymethyl)-2-acetamido-9H-purine (2.05 g, 7 mmole) was dissolved in water (25 ml), 40 % aqueous methylamine (12.5 ml) was added and the solution was stirred at room temperature for 10 minutes. The reaction mixture was evaporated to dryness with successive addition of water and ethanol. The crude product was recrystallized from 100 % ethanol with the addition of activated charcoal to give deoxyacyclovir (1.16 g, 79.5 %) as white crystals, m.p. 192.5-193°C, which is identical with the product obtained in method A.

## Claims

1. Process for preparing purine derivatives of the formula I 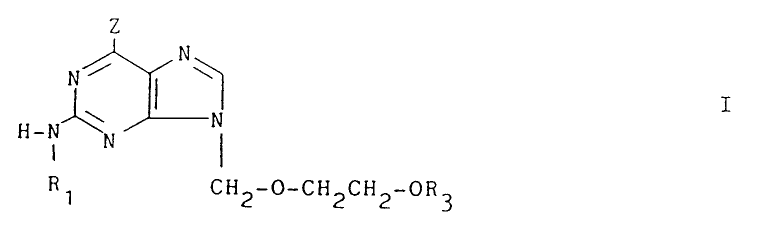 wherein
R₁ represents hydrogen, CH₃CO or a higher acyl,
R₃ represents hydrogen, CH₃CO or a higher acyl, CF₃CO, CCl₃CO, PhCO or CH₂ Ar, such as benzyl or substituted benzyl, on benzoisothiazolyl-S,S-dioxide, and
Z represents OH, hydrogen or halo,
wherein the following compounds are excluded:
(i) Z is OH, R₁ is CH₃CO or higher acyl, and R₃ is hydrogen,
(ii) Z is hydrogen, R₁ is CH₃CO or higher acyl, and R₃ is hydrogen, and
(iii) Z is halo, R₁ is CH₃CO or higher acyl, and R₃ is hydrogen
characterized in that
a) in the first variant for preparing compounds of the formula I, wherein Z represents halo or hydrogen and R₁ is hydrogen, a protected acyclovir of the formula II 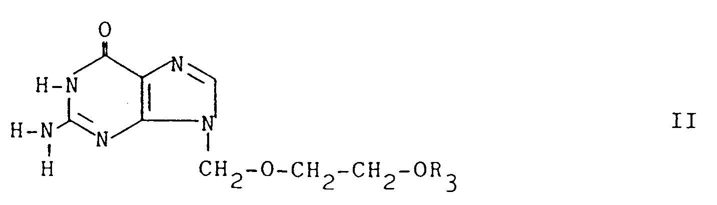 wherein R₃ has the above meanings except hydrogen,
is halogenated to obtain a compound of formula I, wherein Z is halo and R₃ has the above meanings except hydrogen, the obtained compound is hydrogenated and, when R₃ is not CH₂ Ar or substituted aryl, deblocked to compound I with Z = H and R₃= H, or
b) in the second variant for preparing compounds of the formula I, wherein the radicals have the meanings at formula I, a compound of formula III 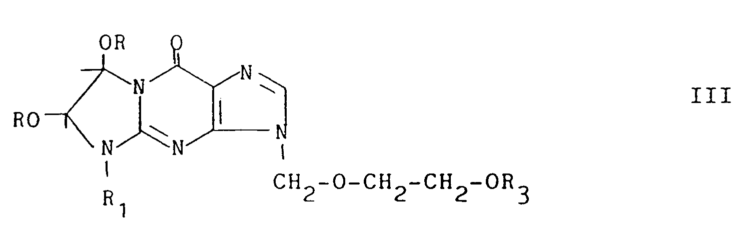 wherein R represents CH₃CO, isobutyryl or higher acyl and R₁ and R₃ have the above meanings except hydrogen,
is selectively deblocked with weak bases or acids or in the presence of a catalyst to a compound of the formula I, wherein Z is OH, R₁ has the meaning of formula I and R₃ has the meaning of formula I except hydrogen, which compound is optionally reacted as in the process variant a), i.e. the obtained compound is halogenated to obtain a compound of the formula I, wherein Z is halo, and the latter is, by hydrogenation or photochemical dehalogenation, optionally converted to compound I, wherein Z is hydrogen.

2. Process for preparing 9-(2-acetoxyethoxymethyl)-2-amino-6-chloro-9-H-purine, characterized in that 9-(2-acetoxyethoxymethyl)guanine is chlorinated.

## Patentansprüche

1. Verfahren zur Herstellung von Purinverbindungen der Formel I 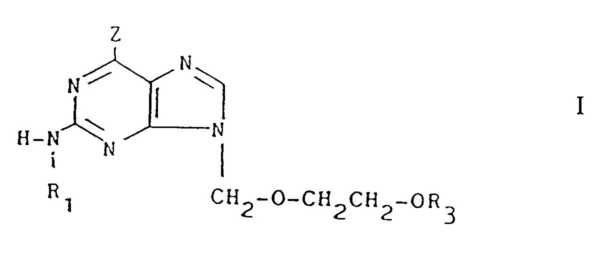 worin
R₁ Wasserstoff, CH₃CO oder höheres Acyl,
R₃ Wasserstoff, CH₃CO oder höheres Acyl, CF₃CO, CCl₃CO, PhCO oder CH₂Ar wie Benzyl oder substituiertes Benzyl, oder Benzoisothiazolyl-S,S-Dioxid, und
Z OH, Wasserstoff oder Halogen bedeuten,
wobei die folgenden Verbindungen ausgeschlossen sind:
(i) Z ist OH, R₁ ist CH₃CO oder höheres Acyl und R₃ ist Wasserstoff,
(ii) Z ist Wasserstoff, R₁ ist CH₃CO oder höheres Aryl und R₃ ist Wasserstoff, und
(iii) Z ist Halogen, R₁ ist CH₃CO oder höheres Acyl und R₃ ist Wasserstoff,
*dadurch gekennzeichnet, daß*
a) gemäß der ersten Variante zur Herstellung der Verbindungen der Formel I, worin Z Halogen oder Wasserstoff bedeutet und R₁ Wasserstoff ist, ein geschütztes Acyclovir der Formel II 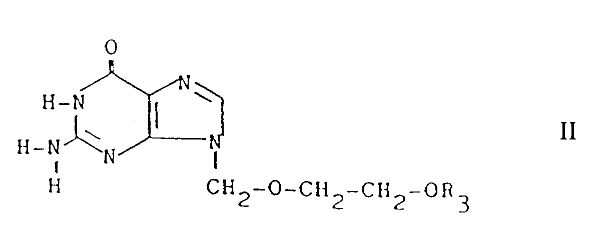 worin R₃ die oben angegebenen Bedeutungen außer Wasserstoff hat,
halogeniert wird, um eine Verbindung der Formel I zu erhalten, worin Z Halogen ist und R₃ die oben angegebenen Bedeutungen außer Wasserstoff hat, die erhaltene Verbindung hydriert wird und, wenn R₃ nicht CH₂Ar oder substituiertes Aryl ist, zur Verbindung I mit Z = H und R₃ = H deblockiert wird, oder
b) gemäß der zweiten Variante zur Herstellung der Verbindungen der Formel I, worin die Radikale die Bedeutungen aus der Formel I haben, eine Verbindung der Formel III 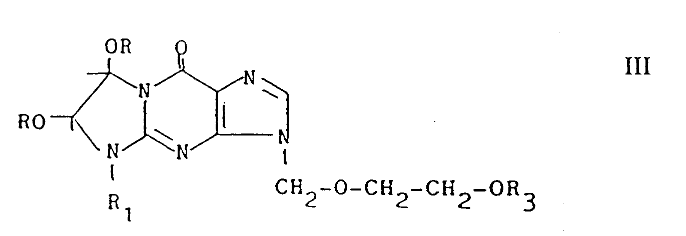 worin R CH₃CO, Isobutyryl oder höheres Acyl bedeutet und R₁ und R₃ die oben angegebenen Bedeutungen außer Wasserstoff haben,
mit schwachen Basen oder Säuren oder in der Anwesenheit eines Katalysators zu einer Verbindung der Formel I, worin Z OH ist, R₁ die Bedeutung aus der Formel I hat und R₃ die Bedeutung aus der Formel I außer Wasserstoff hat, selektiv deblockiert wird, welche Verbindung gegebenenfalls wie in der Verfahrensvariante a) umgesetzt wird, also die erhaltene Verbindung halogeniert wird, um eine Verbindung der Formel I, worin Z Halogen ist, zu erhalten, und die letztere gegebenenfalls durch Hydrierung oder photochemische Dehalogenierung zur Verbindung I, worin Z Halo ist, überführt wird.

2. Verfahren zur Herstellung von 9-(2-Acetoxyethoxymethyl)-2-amino-6-chloro-9-H-purin, *dadurch gekennzeichnet,* daß 9-(2-Acetoxyethoxymethyl)guanin chloriert wird.

## Revendications

1. Procédé pour préparer des dérivés de purine de formule I 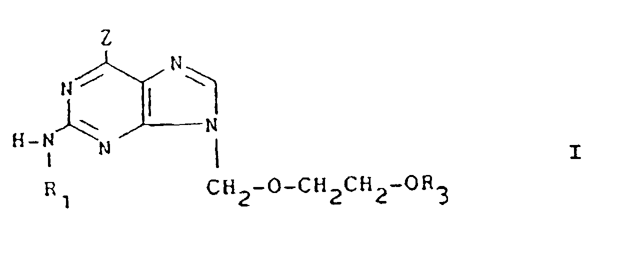 dans laquelle
R₁ représente l'hydrogène, CH₃CO ou un acyle supérieur,
R₃ représente l'hydrogène, CH₃CO ou un acyle supérieur, CF₃CO, CCl₃CO, PhCO ou CH₂ Ar, tel que le benzyle ou la benzyle substitué, ou le benzoisothiazolyl-S,S-dioxide, et
Z représente OH, l'hydrogène ou un halogène,
dans laquelle les composés suivants sont exclus :
(i) Z est OH, R₁ est CH₃CO ou un acyle supérieur, et R₃ est l'hydrogène,
(ii) Z est l'hydrogène, R₁ est CH₃CO ou un acyle supérieur, et R₃ est l'hydrogène, et
(iii) Z est un halogène, R₁ est CH₃CO ou un acyle supérieur, et R₃ est l'hydrogène
caractérisé en ce que
a) dans la première variante pour préparer les composés de formule I, où Z représente un halogène ou l'hydrogène et R₁ l'hydrogène,
un acyclovir protégé de formule II 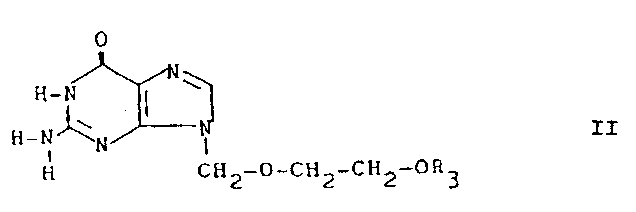 dans laquelle R₃ a les acceptations ci-dessus excepté l'hydrogène,
est halogéné pour obtenir un composé de formule I, où Z est un halogène et R₃ a les accceptations ci-dessus excepté l'hydrogène, le composé obtenu est hydrogéné et, lorsque R₃ n'est pas CH₂ Ar ou un aryle substitué, débloqué en composé I avec Z = H et R₃ = H, ou
b) dans la seconde variante pour préparer les composés de foraule I, où les radicaux ont les acceptations de la formule I,
un composé de formule III 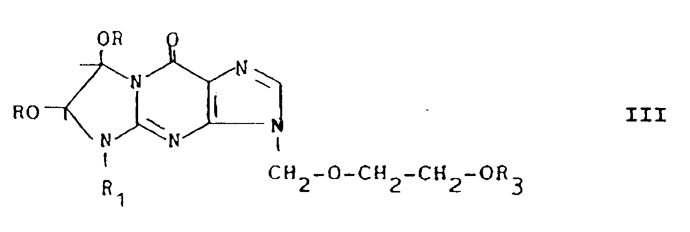 dans laquelle R représente CH₃CO, l'isobutyryle ou un acyle supérieur et, R₁ et R₃ ont les acceptations ci-dessus excepté l'hydrogène,
est sélectivement débloqué avec des bases ou des acides faibles ou en présence d'un catalyseur en un composé de formule I, où Z est OH, R₁ a les acceptations de la formule I et R₃ a les acceptations de la formule I excepté l'hydrogène, lequel composé est facultativement mis à réagir comme dans la variante a) du procédé, c'est-à-dire que le composé obtenu est halogéné pour obtenir un composé de formule I, où Z est un halogène, et ce dernier est, par hydrogénation ou déhalogénation photochimique, facultativement converti en composé I, dans lequel Z est l'hydrogène.

2. Procédé pour préparer la 9-(2-acétoxyéthoxyméthyl)-2-amino-6-chloro-9-H-purine, caractérisé en ce que la 9-(2-acétoxyéthoxyméthyl)guanine est chlorée.
